# EUROPEAN PATENT SPECIFICATION

(11) **EP 4 330 968 B1**
(45) Date of publication and mention of the grant of the patent: **21.10.2026**
(21) Application number: 22722389.8
(22) Date of filing: 28.04.2022
(51) Int. Cl.: G16B 15/30, G06V 20/69, G06V 10/82, G06V 10/46, G06F 18/22, G06F 18/25, G06N 3/0464, G06N 3/0895, G06N 3/09

(54) **MOLECULE IDENTIFICATION AND CLASSIFICATION USING MOLECULAR SURFACE PROPERTIES**
IDENTIFIZIERUNG UND KLASSIFIZIERUNG VON MOLEKÜLEN UNTER VERWENDUNG MOLEKULARER OBERFLÄCHENEIGENSCHAFTEN
IDENTIFICATION ET CLASSIFICATION DE MOLÉCULES AU MOYEN DE PROPRIÉTÉS DE SURFACE MOLÉCULAIRE

(30) Priority: 29.04.2021 US 202163181772 P; 27.08.2021 EP 21315150
(43) Date of publication of application: 06.03.2024
(73) Proprietor: Sanofi, 75017 Paris (FR)
(72) Inventor: RIAHI SAMANI, Saleh, Bridgewater, New Jersey 08807 (US); WEI, Shuai, Bridgewater, New Jersey 08807 (US); QIU, Yu, Bridgewater, New Jersey 08807 (US); ZHOU, Yanfeng, Bridgewater, New Jersey 08807 (US); LEE, Jae Hyeon, Bridgewater, New Jersey 08807 (US)
(74) Representative: McDougall, James
(86) International application number: PCT/US2022/026761
(87) International publication number: WO 2022/232412

(56) References cited:
- GAINZA P ET AL: "Deciphering interaction fingerprints from protein molecular surfaces using geometric deep learning", NATURE METHODS, NATURE PUBLISHING GROUP US, NEW YORK, vol. 17, no. 2, 9 December 2019 (2019-12-09), pages 184 - 192, XP037006735, ISSN: 1548-7091, [retrieved on 20191209], DOI: 10.1038/S41592-019-0666-6
- TORNG WEN ET AL: "High precision protein functional site detection using 3D convolutional neural networks", BIOINFORMATICS, vol. 35, no. 9, 1 May 2019 (2019-05-01), GB, pages 1503 - 1512, XP055947498, ISSN: 1367-4803, Retrieved from the Internet <URL:https://academic.oup.com/bioinformatics/article-pdf/35/9/1503/28557284/bty813.pdf> [retrieved on 20220730], DOI: 10.1093/bioinformatics/bty813
- YIN SHUANGYE ET AL: "Fast screening of protein surfaces using geometric invariant fingerprints", PROCEEDINGS OF THE NATIONAL ACADEMY OF SCIENCES, vol. 106, no. 39, 29 September 2009 (2009-09-29), pages 16622 - 16626, XP055947577, ISSN: 0027-8424, DOI: 10.1073/pnas.0906146106

## Description

### BACKGROUND

Protein can be represented in different forms, such as in a sequence, in a 3D structure, or in its molecular surface. There are many methods developed for sequence and structure comparison. Molecular surface comparison methods have not been extensively studied compared to those of sequence and structure.

Gainza P et al: "Deciphering interaction fingerprints from protein molecular surfaces using geometric deep learning", Nature Methods, Nature Publishing Group US, New York, vol. 17, no. 2, 9 December 2019, pages 184-192, propose that molecular surfaces are fingerprinted with patterns of chemical and geometric features that reveal information about the protein's interactions with other biomolecules. The central hypothesis is that proteins with no sequence homology that undergo similar biomolecular interactions may display similar patterns, which are difficult to grasp by visual analysis but could be learned from largescale datasets. They present MaSIF (molecular surface interaction fingerprinting), a general geometric deep learning method to recognize and decipher patterns on protein surfaces, without explicit consideration of the underlying protein sequence or structural fold.

### SUMMARY

Implementations of the present disclosure include computer-implemented methods and systems for identification and classification of molecules, such as protein molecules, by using surface characteristics of the molecules. The implementations describe a geometry-aware system capable of performing two complementary methods to identify or classify a target molecule. These methods apply deep learning and binned-feature matrices to generate numerical representations of molecular surfaces of customizable size from geometric and chemical features of the surfaces. The numerical representations are called "surface IDs" herein. The methods then use these surface IDs to search for similar surfaces, for example, among known molecules. Examples of applications of these methods are provided herein to (i) identify a target protein molecule by comparing the surface of the molecule to surfaces of known epitopes, and (ii) cluster antibodies by their functional paratopes.

In some implementations, the present method include: receiving, by a system of one or more computers, a target molecule to be identified or classified; identifying, by the system, a surface mesh that defines a surface of the target molecule, the surface mesh comprising a plurality of vertices; identifying, by the system, a plurality of surface patches by associating each vertex of the surface mesh with a respective patch; generating, by the system, a respective latent space ID for each of the surface patches by using a neural network; generating, by the system, a respective real space ID for each patch in the surface patches by using a radial distribution of one or more geometric or chemical features of the patch; obtaining, by the system and from a storage medium, one or more candidate items with known surfaces; using, by the system, the latent space IDs and the real space IDs to identify at least one candidate item that includes a surface resembling a surface region of the target molecule, wherein the surface region comprises multiple patches in the plurality of surface patches of the target molecule; using the at least one candidate item to determine an identification or a classification of the target molecule; and providing, by the system, the identification or the classification of the target molecule for presentation to a user.

The present disclosure also provides one or more non-transitory computer-readable storage medium coupled to one or more processors and having instructions stored thereon which, when executed by the one or more processors, cause the one or more processors to perform operations in accordance with implementations of the methods provided herein.

The present disclosure further provides a system for implementing the methods provided herein. The system includes one or more processors, and a computer-readable storage medium coupled to the one or more processors having instructions stored thereon which, when executed by the one or more processors, cause the one or more processors to perform operations in accordance with implementations of the methods provided herein.

Methods in accordance with the present disclosure may include any combination of the aspects and features described herein. That is, methods in accordance with the present disclosure are not limited to the combinations of aspects and features specifically described herein, but also include any combination of the aspects and features provided.

Among other advantages, the present implementations provide the following benefits. The present implementations focus on the molecular surface features, such as geometrical and chemical features to identify or classify a target molecule. Molecular surfaces are particularly important to identify functions and biomolecular interactions of protein molecules. Diverse functions of proteins are achieved by three-dimensional structures, which are in turn determined by their genetically encoded amino acid sequences. Many methods have been developed to compare and classify proteins using their overall sequence and structural similarities. These types of methods have limitations because overall sequence and structure similarities don't necessarily link to similarities in function. The protein functions are directly resulted from the local structural elements, such as biochemical properties and geometric shape, instead of overall sequence and structure scaffold. The present disclosure provides methods that study and compare such function-related local surface structural features. Local surface is addressed herein as surface "patch" or "region".

Further, the present disclosure provides techniques to improve computational efficiency and speed, and thus reduce the hardware that may be needed to perform 3D analysis of molecular surfaces. Despite its importance, molecular surface comparison methods have not been extensively studied compared to those of sequence and structure. One reason is the degree of freedom for matching 3D objects lacking signature elements, such as secondary structures for the structural alignment. To find local structural similarities, 3D objects must undergo extensive rotational and translational transformations so that various local alignments may be sampled, and differences can be measured. Such transformations impose a large overhead for computational methods. Several algorithms were reported to overcome the spatial degrees of freedom issue and computational complexity, such as geometric hashing, Fourier transformation (FT), 3D Zernike descriptors, and geometric invariant fingerprint descriptors. However, these methods rely on human crafted descriptors and parameters based on heuristics, which may not be optimal in capturing the full complexity of molecular surfaces.

Additionally, the present disclosure provides two complementary methods that can be used in parallel or in sequence to study the same molecular surface. The two methods are performed independently, and their results can be used as complementary to each other, or to verify each other. The first method is a latent space method that uses machine learning algorithms and neural networks to identify known surface regions on a target molecule. The second method uses radial distribution of the surface features and properties of the target molecule to identify a known item, e.g., an epitope or a molecule, with similar surface features and properties.

The details of one or more implementations of the present disclosure are set forth in the accompanying drawings and the description below. Other features and advantages of the present disclosure will be apparent from the description and drawings, and from the claims.

### DESCRIPTION OF DRAWINGS

FIG. 1 depicts an example system that can be used to execute implementations of the present disclosure.
FIGs. 2A and 2B illustrate example processes that are used by an identification module according to the present disclosure.
FIG. 3 is an example process that can be executed in accordance with implementations of the present disclosure.
FIG. 4 shows a schematic diagram of an example computing device and a mobile computing device that can perform the methods described in the present disclosure.
FIG. 5 is an example alignment between vertices of a target molecule and an epitope.
FIG. 6 shows an example result in identifying two surface regions of a target molecule based on a known epitope.
FIGs. 7A and 7B are example classifications of paratopes and epitopes according to the present disclosure.
FIGs. 8A and 8B depict the performance of the present techniques in identifying test molecules.

Like reference symbols in the various drawings indicate like elements.

### DETAILED DESCRIPTION

The present implementations provide systems to identify or classify a target molecule or its properties. The systems are capable of performing two complementary methods to compare molecular surfaces in real space and in latent space for identifying the surfaces' similarities. The method that uses real space comparisons assigns a "real space surface ID" to the target molecule, and the method that uses latent space comparisons assigns a "latent space surface ID" to the target molecule. The system uses these surface IDs to classify or identify the target molecule by comparing the surface IDs to a set of known surface IDs associated with known molecules.

In an example application, the target molecule can be a protein molecule. The system can identify an unknown protein by generating and using one or more surface IDs for surface patches of the protein's surface. For example, the system can receive an unknown antigen, find a known epitope with a surface similar to surface region of the antigen, and classify or identify the unknown antigen with respect to the identified epitope. The found epitope can be used to identify or to engineer a paratope that would match the unknown antigen through the surface region that is similar to the antigen's surface.

Using the real space and latent space methods, the system may obtain multiple identifications or classifications for the same target molecule. For example, the system may find one identification for each of the latent and real space IDs associated with the target molecule, or one identification for different surface regions of the molecule. Considering that functions and biomolecular interactions of molecules such as proteins are defined by their local surfaces (i.e., surface regions), the system may present all of the obtained identification and classifications to a user, for example, for further engineering of the molecule. Additionally, or alternatively, the system may use the obtained identifications and classification to verify its accuracy in identifying or classifying the target molecule, for example, by comparing an overlap between the identified molecules or classes.

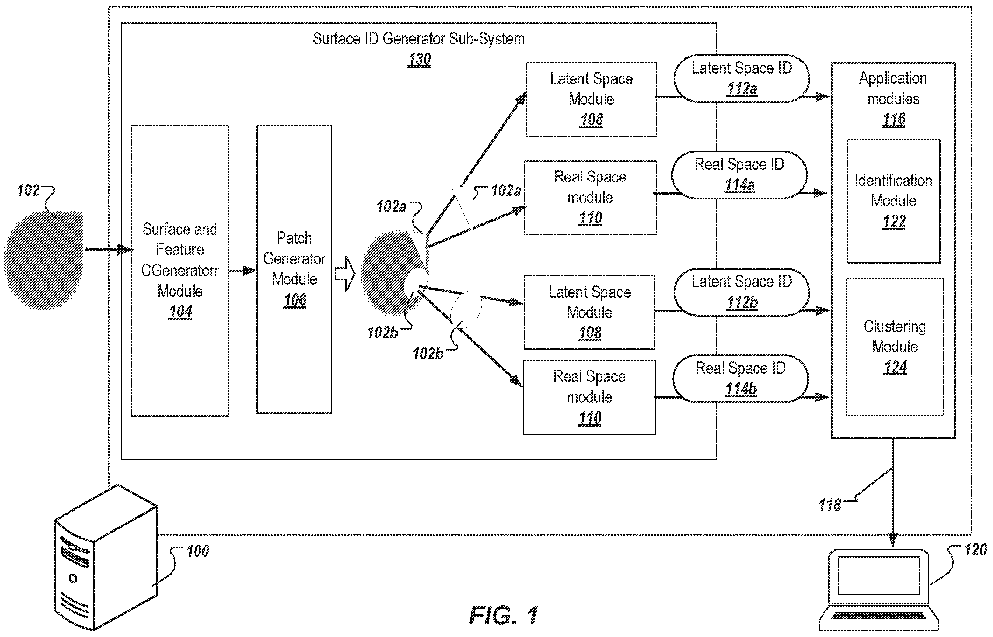

FIG. 1 illustrates an example system 100 according to implementations of the present disclosure. System 100 receives target molecule 102, analyzes the target molecule, and provides (118) an identification or a classification of the target molecule for presentation, for example, on a display of presentation device 120.

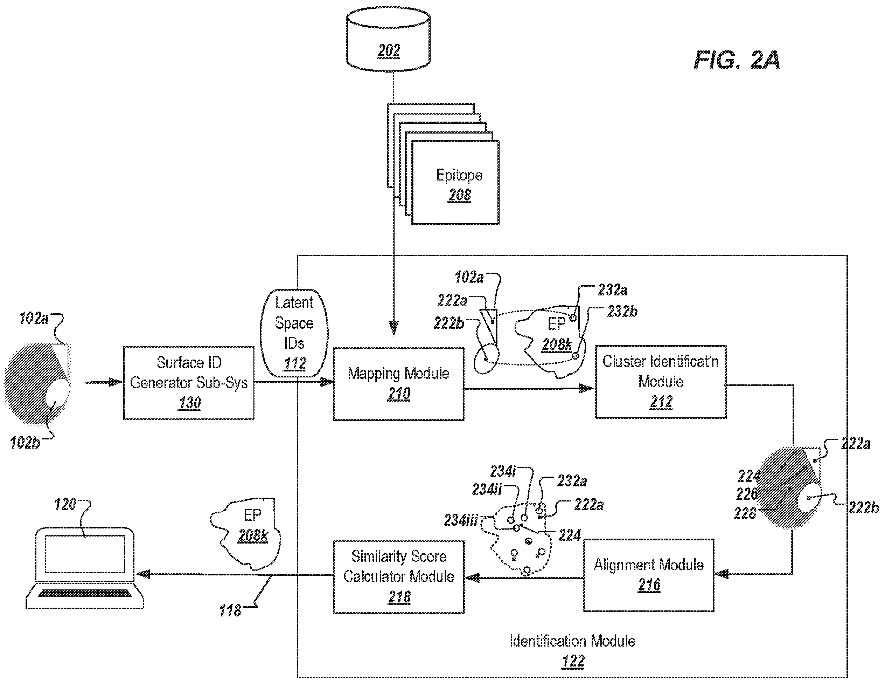

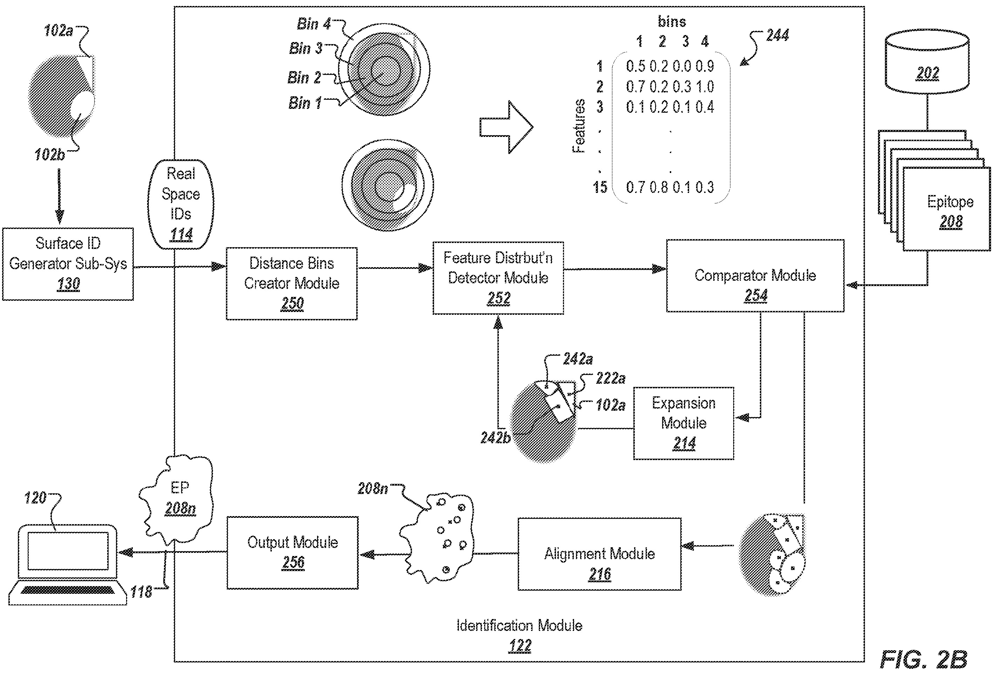

System 100 includes surface ID generator sub-system 130 and one or more application modules 116. Surface ID generator sub-system 130 is capable of producing latent space IDs and real space IDs for surface patches of target molecule 102's surface. The application modules 116 receives the IDs and provides an identification or a classification of target molecule 102 based on those IDs. Details about application modules 116 are described below with respect to FIGs. 2A and 2B.

Surface ID generator sub-system 130 includes multiple modules: surface and feature generator module 104, patch generator module 106, latent space module 108, and real space module 110. Latent space module 108 generates latent space IDs and real space module 110 generates real space IDs for target molecule 102's surface.

Surface and feature generator module 104 computes a surface mesh that defines a surface of target molecule 102. The surface mesh includes multiple vertices. The module 104 also computes molecule features associated with each vertex. A feature of a vertex can be a geometric feature such as a shape index, a distance-dependent curvature, or any other feature associated with the geometric position of that vertex on target molecule 102. A feature of a vertex can be a chemical feature such as a hydropathy, a continuum electrostatics, number of free electrons or protons, or any other chemical features of target molecule 102 at that vertex.

Surface and feature generator module 104 sends the identified surface (i.e., the identified vertices) and features associated with each vertex to patch generator module 106. Patch generator module 106 generates patches on the surface mesh. In some implementations, patch generator module 106 generates the patches by identifying surface areas surrounding each of the vertices. In some implementations, the patches have the same radius, or have the same size. For example, each patch can be defined by a corresponding border line that is at a certain geodesic distance from a center vertex of the patch on target molecule 102's surface. The geodesic distance can be a predetermined value, e.g., a value between 5 to 20 angstroms (Å). The geodesic distance can be preselected based on the applications, e.g., the type of a target classification, or based on the trained samples used to identify or classify target molecule 102. In some implementations, a patch can include multiple vertices (e.g., 50 vertices) that are each within the geodesic distance from the center vertex of the patch. In some implementations, each vertex on the surface mesh of target molecule 102 is the center vertex of a respective patch. Two or more patches can have different shapes.

FIG. 1 shows two patches 102a and 102b that patch generator module 106 has generated on the surface of target molecule 102. Patch generator module 106 provides each of the generated patches 102a, 102b to latent space module 108 and real space module 110.

Latent space module 108 generates a respective latent space ID 112a, 112b for each of the generated patches 102a, 102b by using a neural network. The neural network can have multiple layers optimized for the patch sizes. Using the same size patches would beneficially allow using the same neural networks, i.e., the same layers, on different patches, and thus, would reduce computational complexity that may be needed to optimize the layers if the patches had different sizes. The layers can include convolutional, Gaussian Error Linear Unit (GeLU), or any other layers that would be beneficial in identifying a surface patch.

The neural network can be trained in a supervised, a semi-supervised, or an unsupervised fashion. The training is performed to minimize distance of similar patches on training data. In an example process, a single vertex of a molecular surface mesh is chosen at random during each mini-batch step of the training. In a surface patch, all vertices that are within a predetermined distance from each other are considered as "positive" pairs. For example, within the 12 Å radius surface patch or region associated with a chosen vertex, all pairs of patches with their center vertices that are within 1.5 Å in geodesic distance of each other are considered "positive" pairs and those outside 5.0 Å "negative" pairs, with all other pairs being excluded. Adam optimizer can then be used with default parameters for specific learning rate (e.g., learning rate of 5 × 10⁻⁴), and decoupled weight decay with specific penalty (e.g., 1 × 10⁻²).

Real space module 110 generates a respective real space ID 114a, 114b for each of the generated patches 102a, 102b by using the features that the surface and feature generator module 104 computed for the respective vertices of the patches. Real space module 110 generates each of the real space IDs (e.g., real space ID 114a) by using radial distribution of the features on the respective patches (e.g., patch 102a) of the target molecule 102, and by comparing the distribution to predefined rules that the system uses to create the real space IDs.

Compared to latent space module 108, real space module 110 provides more flexibility in using separate features or weighted feature combinations for specific tasks (e.g., for different classifications, or for identifying specific epitopes) because the real space module 110 does not use a pre-trained neural networks and is not limited to specific patch sizes. In general, the real space module 110 generates a real space ID of a target patch by performing three main steps: step 1: obtaining known patches or regions on known surfaces with feature distributions similar to the feature distribution on the target patch, step 2: obtaining averaged feature similarity scores based on vertices within 2.5 angstrom of the central vertex of the target patch, and step 3: aligning top patches (e.g., top 10% or top 200 patches with the most precise alignment) obtained from step 2 and characterizing the target patch based on based on the alignment scores of the patches that are similar to the target patch.

Latent space module 108 and real space module 110 send the respective latent space and real space IDs for each patch to application modules 116. The application modules 116 use the IDs to identify or classify the target molecule 102. As explained earlier, since application modules 116 receive multiple patches and multiple IDs for the patches, the application modules 116 may provide multiple identifications or classes for the target molecule 102.

Application modules 116 include identification module 122 and clustering module 124. FIGs. 2A and 2B are block diagrams showing example processes that identification module 122 can perform to identify a target molecule, for example, 102. The identification module 122 shown in FIG. 2A provides an identification based on the latent space IDs 112 of the target molecule 102. The identification module 122 shown in FIG. 2B provides an identification based on real space IDs 114 of the target molecule 102.

In the examples shown in FIGs. 2A and 2B, the target molecule 102 is an antigen with unknown properties. The output of the identification module 122 are epitopes 208k and 208n that the module 122 has identified as having surfaces or properties similar to respective surface regions of the antigen - i.e., target molecule 102. The surface regions cover the patches 102a and 102b. Epitope 208k can be the same as or different from epitope 208n.

Referring to FIG. 2A, the identification module 122 identifies an epitope - i.e., epitope 208k - with a surface similar to a target "surface region" (i.e., a portion) of target molecule 102's surface that covers patches 102a and 102b. To do so, the identification module 122 receives the latent space IDs 112a, 112b of the patches 102a, 102b from the surface ID generator sub-system 130 and analyzes those patches through multiple modules: mapping module 210, cluster identification module 212, alignment module 216, and similarity score calculator module 218.

Mapping module 210 uses the latent space IDs 112a, 112b to identify one or more candidate epitopes that include respective surfaces resembling patches 102a, 102b on the target molecule. To do this, mapping module 210 communicates with a storage device 202 to obtain stored epitopes 208. The stored epitopes 208 can have surfaces with known properties or features. Each of the stored epitopes has a respective real space ID and a respective latent space ID that is computed and stored for the epitope.

Mapping module 210 tries to map the vertices in patches 102a, 102b to respective vertices on surfaces of the stored epitopes 208, and identifies, from among the stored epitopes 208, one or more candidate epitopes that have vertices mappable to the patches' vertices. In some implementations, mapping module 210 identifies, from among stored epitopes 208, one or more epitopes that each has a respective surfaces similar to at least one of the patches 102a, 102b on target molecule 102. For example, mapping module 210 identifies epitope 208k as a candidate epitope because the module can map vertices 222a and 222b of patches 102a and 102b, to vertices 232a and 232b on the surface of epitope 208k. Accordingly, an epitope can be a mapped for one or for multiple patches of the target molecule, and a patch on a target molecule can be mapped to more than one epitope.

In some implementations, a first vertex on the target molecule's patches is mapped to a second vertex on an epitope when a difference between an ID vector distance at the first vertex and at the second vertex is within a specific threshold. The ID vector distance at a vertex is a vector that includes values calculated for all features at that vertex. The features can include geometrical or chemical feature, for example, any of the features discussed earlier, or a combination of geometrical and chemical features.

Given that most useful applications are likely to prioritize high recall rate, the comparison made by mapping module 210 with a permissive threshold will in general produce many dissimilar vertex pairs on target molecule and the candidate epitopes, e.g., epitope 208. Further, for many applications, it is not the discovery of two similar patches of a fixed radius (e.g., 6 Å) centered at two different vertices that would be of interest, but instead it is the discovery of relatively larger similar surface regions. Cluster identification module 212, alignment module 216, and similarity score calculator module 218 find and verify existence of those larger similar surface regions. A "surface region" can thus cover multiple patches and is larger than any of the patches that the surface region covers.

The mapping module 210 sends the identified candidate epitopes, such as epitope 208k, to cluster identification module 212. Cluster identification module 212 identifies a cluster of vertices on target molecule 102, where each vertex in the cluster is within a predetermined threshold distance from at least one of the mapped vertices on the patches 102a, 102b, i.e., vertices 222a, 222b. For example, cluster identification module 212 identifies vertices 224, 226, and 228 on target molecule 102 as vertices that would each fit within a predetermined distance from at least one of vertices 222a and 222b of patches 102a and 102b. Surface patches on target molecule 102 that cover the cluster of vertices 222a, 222b, 224, 226, and 228 define a surface region.

Cluster identification module 212 sends the identified cluster of vertices to alignment module 216. Alignment module 216 aligns the cluster's vertices with vertices on the candidate epitope 208k to verify a 3-dimensional ("3D") alignment of a surface region of target molecule 102 to vertices (e.g., 234i, 234ii, 234iii) on the candidate epitope 208k's surface.

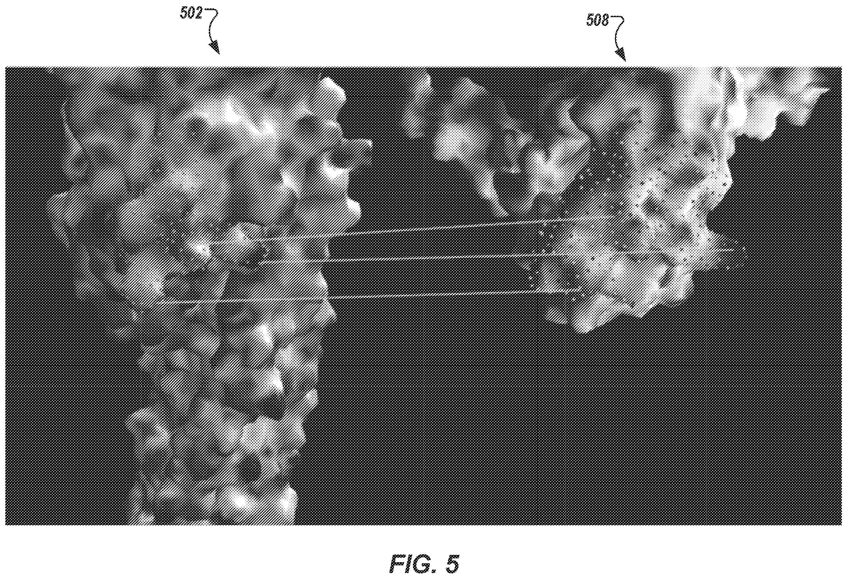

Alignment module 216 can perform the alignment by using gradient descent on the cluster's vertices with the objective of minimizing 3D Euclidean distance of hit vertex pairs by rotating and translating the target surface region. To initialize the alignment, alignment module 216 subtracts the respective geometrical centroids of surface regions from the vertices and uses 3D rotations uniformly sampled on a unit sphere. Further, the aligned molecules can be visually inspected, e.g., by an operator, to verify an accurate alignment. An example alignment is shown in FIG. 5, where vertices on target molecule 502 are aligned with vertices on known epitope 508.

For each of the candidate epitopes, e.g., epitope 208k, similarity score calculator module 218 quantifies the degree of similarity between the surface region of target molecule 102 and the surface of candidate epitope (after alignment). In some implementations, similarity score calculator module 218 calculates a spatial similarity score *(SSS)* as a weighted mean input feature distance between pairs of vertices that are within a 3D distance cutoff (e.g., 1.5 Å) to determine the degree of similarities, for example, by using the following formula: $SSS = \frac{{\sum }_{ij} {W}_{ij} \left|{F}_{i}-{F}_{j}\right|}{{\sum }_{ij} {W}_{ij}} + \frac{{\sum }_{ij} {W}_{ji} \left|{F}_{i}-{F}_{j}\right|}{{\sum }_{ij} {W}_{ji}}$ (1) where ${W}_{ij} = Softmax \left({{r}_{ij}}^{2}/{sig}^{2}\right)$ (2) over *j,* Fi and Fj are feature vectors of points *i* and j and *rᵢⱼ* is 3D distance between points *i* and *j*, and *sig* is fixed at 0.25.

Similarity score calculator module 218 provides (118) each candidate epitope that has a degree of similarity (i.e., *SSS*) higher than a threshold score, as an epitope with a surface resembling at least a portion of target molecule 102's surface (i.e., a surface region).

Although the description above focuses on one cluster of vertices, cluster identification module 212 can identifying multiple clusters of vertices on target molecule 102 with vertices mapped to vertices of the same candidate epitope, e.g., epitope 208k. Modules 216 and 218 can perform the same procedure on each of those clusters to identify candidate epitopes with surfaces that resemble at least a respective surface region of target molecule 102's surface. To improve the process efficiently and reduce computational burdens, the identification module 122 can filter out clusters that have less than a predetermined number of vertices, e.g., 40 vertices, and perform the alignment on the remaining clusters.

In some implementations, cluster identification module 212 reduces the number of vertex pairs that are to be aligned at alignment module 216 to reduce the computational burdens and improve the process efficiency. For example, cluster identification module 212 can rank the clusters that it has identified, and filter out clusters that are ranked lower than a specific threshold rank. A cluster can be ranked, for example, based on one or more parameters such as: number of mapped vertices in the cluster, a ratio of number of mapped vertices in the cluster to a total number of vertices in the cluster, number of vertices on a candidate item mapped to one or more vertices of the cluster, or a ratio of number of vertices on a candidate item mapped to one or more vertices of the cluster, to a total number of vertices on the candidate item. These parameters would indicate the density of alignable pairs of vertices because good matches between vertices on target molecule 102 and a candidate epitope would share a large number of vertices in compact surface regions.

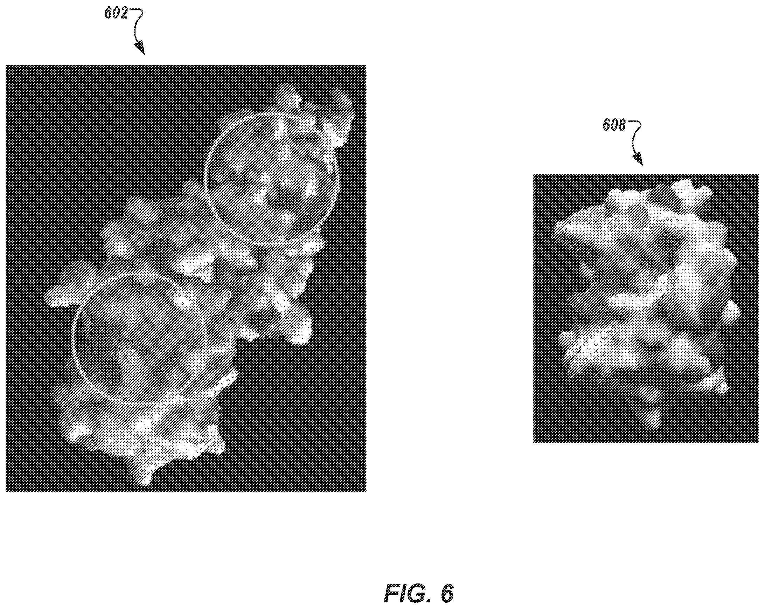

FIG. 6 shows an example result in identifying a surface region of a target molecule 602 based on a known epitope 608, according to implementations of the present disclosure. Two candidate surface regions (i.e., a top and a bottom surface regions) are identified on target molecule 602. Due to the similarities between the bottom surface region on target molecule 602 and surface of epitope 608, the known properties of epitope 608 can be used to recognize parameters or unknown features of the target molecule 602, for example, to engineer an antibody for the target molecule based on paratopes or antibodies that are known for the epitope 608.

While identification module 122 uses the latent space IDs 112 of target molecule 102's patches to perform the process shown in FIG. 2A and identify epitope 208k, the module can perform a complementary process using real space IDs 114 of the patches to identify the same or different epitopes as epitopes that resemble at least a portion of target molecule 102's surface. An example of this complementary process is shown in FIG. 2B.

Referring now to FIG. 2B, identification module 122 identifies an epitope - i.e., epitope 208n - with a surface having features similar to a surface region on target molecule 102's surface. Identification module 122 performs this process by using the following modules: distance bins creator module 250, feature distribution detector module 252, comparator module 254, expansion module 214, alignment module 216, and output module 256. The idea underlying the process shown in FIG. 2B is that two surface patches are similar if the radial distribution of the selected features is similar over the two patches so that the two patches can be properly aligned.

To compare radial distribution of a feature on target molecule 102's surface and features on stored epitopes 208, distance bins creator module 250 creates "bins" on the surface of target molecule 102, and feature distribution detector module 252 determines the distribution of one or more features in each bin. The bins can be in form of spheres that are all centered at the same point but have varied geodesic radiuses; for example, see bins 1 through 4 shown in FIG. 2B. The module 250 uses the real space IDs 114 to make an estimate of where each of the patches 102a, 102b is located with respect to the bins. The feature distribution detector module 252 determines the feature distributions in bins 1 through 4 based on the location of the patches 102a, 102b. In the example shown in FIG. 2B, feature distribution detector module 252 determines distribution of fifteen features in each of the bins, which is shown in form of matrix 244.

Feature distribution detector module 252 determines the distribution of each feature within the respective bins. The module 252 can make this determination for each patch and for different features, e.g., the geometric or chemical features described above. Feature distribution detector module 252 can use the real space IDs to determine the feature distributions on the surface of target molecule 102 because the real space IDs 114 of the patches were generated based on the feature distribution on the surface of target molecule 102.

Feature distribution detector module 252 provides the feature distributions to comparator module 254. Comparator module 254 compares the radial feature distributions of target molecule 102 with radial feature distributions on stored epitopes 208 obtained from the storage device 202. This comparison can be carried out through computing the Euclidian distance between the normalized 2D, i.e., radial and feature space, histograms of patches on target molecule and epitopes 208. If the Euclidian distance is lower than a specific threshold, the patches have similar feature distributions.

In an initial round of comparing process, the searching region is limited to patches 102a, 102b that were identified by the surface ID generator sub-system 130. The comparator module 254 searches among the stored epitopes and provides, to expansion module 214, patches that have feature distributions similar to the surface of at least one epitopes. For example, the comparator sends patch 102a to expansion module 214 in response to finding an epitope, e.g., epitope 208n, that has a radial feature distribution similar to the radial feature distribution of patch 102a.

Subsequently, expansion module 214 expands the searching region by identifying neighboring patches that are within a specific distance from the patch(es) identified in the initial round of comparing process, e.g., patch 102a. These neighboring patches are shown in FIG. 2B, and are identified by their respective center vertices 242a and 242b. These neighboring patches can have the same or different sizes compared to each other or compared to patch 102a. The neighboring patches are the nearest neighbor patches that have center vertices located within the specific distance, e.g., within 2.5 Å, from vertex 222a of patch 102a.

Expansion module 214 sends back the neighboring patches to feature distribution detector module 252 to detect radial feature distribution of a surface region that is made of the patch 102a, which was processed in the initial round of comparing process, and its neighboring patches, which were identified by expansion module 214.

In a subsequent round of comparing process, comparator module 254 compares the feature distribution on the surface region (rather than on isolated patches) to feature distribution of stored epitopes 208. Alternatively, comparator module 254 performs the subsequent comparing process only on epitopes that were found as having similarities to one or more patches in the initial round of comparing process. If comparator module 254 finds an epitope, e.g., epitope 208n, that has a radial feature distribution similar to the radial feature distribution of the surface region, the comparator module provides the surface region to alignment module 216. In some implementations, the comparator module 254 performs the subsequent comparing process to find a one-to-one correspondence between (at least some of) the vertices on the surface region and (at least some of) vertices on epitope 208n. In some implementations, the comparator module 254 uses average similarity scores between the similar vertices of the surface region and vertices of epitope 208n to decide whether epitope 208n has a surface that is alignable with the surface region.

In some implementations, alignment module 216 performs the operations discussed above with respect to FIG. 2A, to verify a 3-dimensional ("3D") alignment of the surface region with vertices on the candidate epitopes surfaces, e.g., epitope 208n's surface. Alternatively, or in addition, alignment module 216 can perform a Monte Carlo (MC)-based alignment on the surface region and the epitope 208n's surface, where a penalty function is minimized following rigid body random transformations. The penalty function is constructed based on the square of minimum distance between each point on the candidate and target surfaces using following equation: 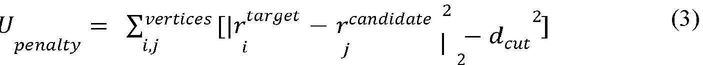 where *U_{penalty}* is the penalty function, $d = \left|{{r}^{target}}_{i}-{{r}^{{candidate}^{2}}}_{j}\right|$ is the minimum distance between vertex *i* on the target molecule and vertex *j* on the candidate epitope, and *d*² *- d_{cut}*² is the penalty for non-overlapping vertices.

Alignment module 216 provides the alignment result, i.e., the alignment score *U_{penalty},* to output module 256. Output module uses the alignment score of each candidate epitope (e.g., epitope 208n) that has been identified as alignable to a surface region of target molecule 102 to decide whether or not send the epitope as output of the identification module 122, for example, for display on presentation device120. The output module 256 may select one or more epitopes with highest alignment scores to be sent as the output.

While FIGs. 2A and 2B show example processes that identification module 122 uses to provide an identification for target molecule 102, clustering module 124 can use similar processes to provide a classification for target molecule 102. Clustering module 124 compares Surface ID (either real space or latent space) of different patches, and cluster them based on their pairwise alignment or similarity scores. For example, antibody paratope regions can be clustered based on their pairwise alignment score, which is corresponding to their functions. For example, clustering module 124 can use the identified epitopes 208k, 208n to identify paratopes that would likely interact with target molecule 102. For example, if the target molecule is a protein for an unknown disease that matches an epitope of influenza, the paratopes used to treat the influenza or other proteins that function similar to the influenza protein - i.e., are classified in the same class with respect to that function - can be used or studied for treatment of the unknown disease.

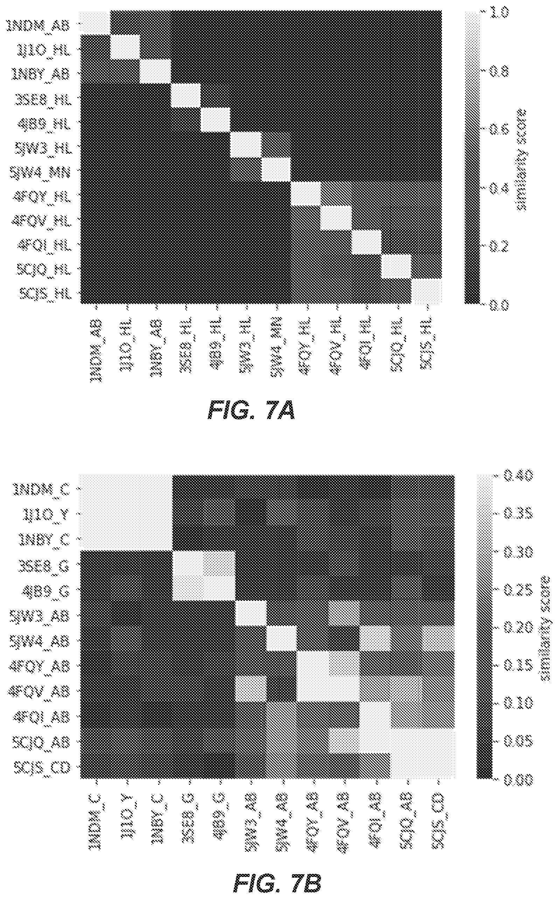

FIGs. 7A and 7B show example classifications of known paratopes and epitopes. If one of these paratopes (or epitopes) is identified as having a similar surface to part of a target molecule, the paratopes or the properties of the paratopes that are in the same class as the identified paratope (or epitope) can be used to study the target molecule. Figure 7A shows pairwise alignment score of antibody paratopes, indicating a group of antibodies with high SSS target similar antigen. Figure 7B shows pairwise alignment score of antigen epitopes, indicating a group of epitopes with high alignment scores are clustered by function. The color shade shows a similarity scores for the paratopes and the epitopes, with the lighter color showing a greater similarity score, as described in the legend of the respective charts.

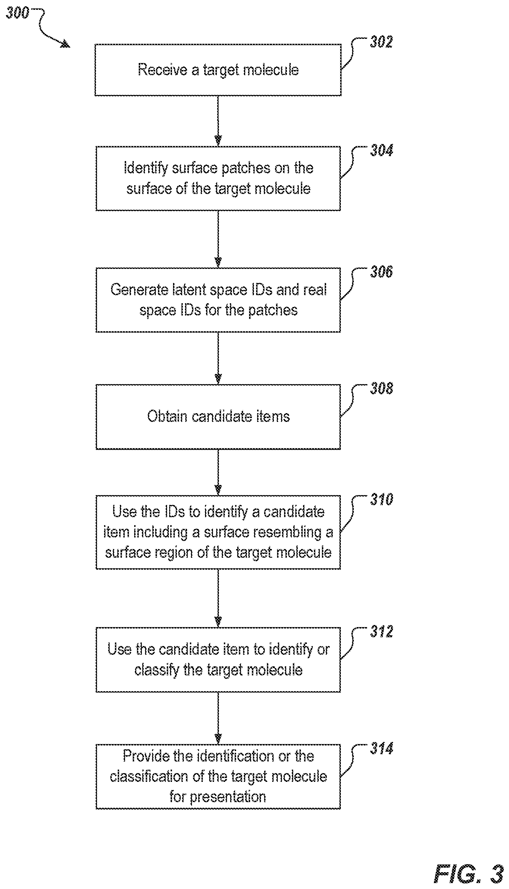

FIG. 3 shows an example process 300 according to implementations of the present disclosure. Process 300 is performed by a computing system, for example, system 100 in FIG. 1.

The system receives a target molecule (302), e.g., target molecule 102 in FIG. 1. The system then identifies surface patches on the surface of the target molecule (304). For example, the system generates a surface mesh on the target molecule's surface, where the surface mesh includes a plurality of vertices. The system then identifies a plurality of surface patches by associating each vertex of the surface mesh with a respective patch.

The system generates a respective latent space ID and a respective real space ID for each of the patches (306). The system can perform the method described with respect to the surface ID generator system 100 to generate the IDs.

The system obtains candidate items (308), and uses the latent space and real space IDs to identify a candidate item that includes a surface resembling a surface region of the target molecule (310). For example, identification module 122 of system 100 (see FIG. 1) communicates with a storage device 202 (see FIGs. 2A, 2B) to obtain epitopes 208 as candidate items. The identification module 122 then uses epitopes 208 to identify epitope 208k, 208n as candidate items that each includes a surface resembling a respective surface region of target molecule 102. The surface region is larger than each of the patches identified in 306.

The system then uses the candidate item identified in 310, to identify or classify the target molecule (312) or to identify a property of the target molecule. For example, system 100 of FIG. 1 identifies a property or a classification of target molecule 102 based on the properties or classifications of the identified epitopes 208k, 208n. The system then provides the identification or the classification of the target molecule for presentation to a user (314), e.g., through a display.

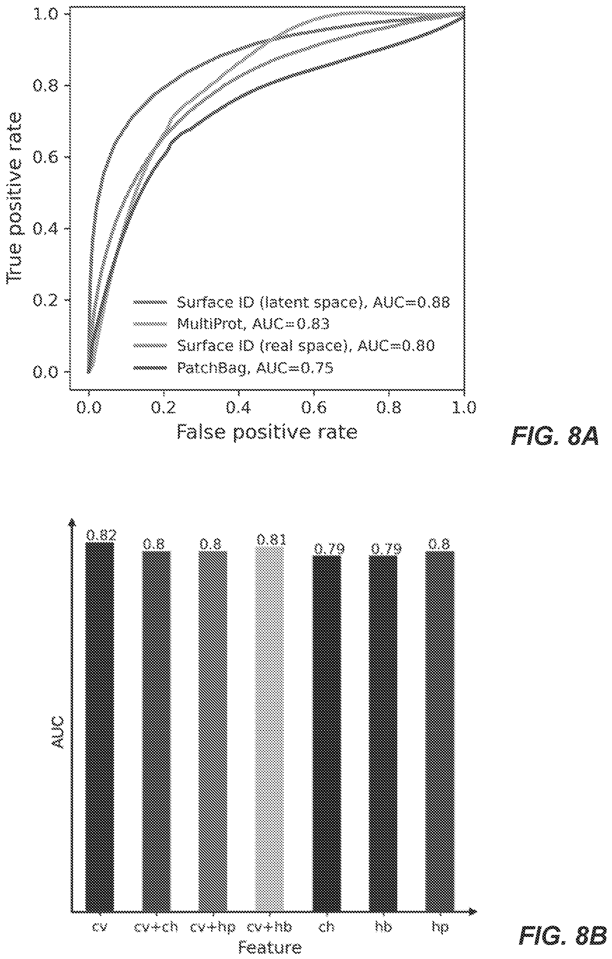

Experiment has shown that the techniques presented in this disclosure are among the top performers used in the industry for surface-based identification/classification of molecules. FIG. 8A shows the performance of the present techniques in identifying test molecules. The figure shows that the true positive rate-i.e., a correct identification of the test molecules-compared to a false positive rate-i.e., an incorrect identification of the test molecules-when the present techniques are used. As shown, an identification/classification based on the latent space and the real space surface IDs, provide AUCs (which stands for area under the curve) as high as 0.88 and 0.8, respectively. Such high AUCs indicate a high accuracies in the results obtained. FIG. 8A also shows the performance of the two other techniques, MultiProt and PatchBag, which are among highly accurate techniques known in the art. As shown, the performance of the present techniques are comparable to MultiProt, and better than PatchBag. The present techniques are also computationally fast. For example, it may take a few seconds or only a few minutes to identify a target molecule by using the present techniques.

Experiment has shown that the choice of chemical and geometrical features for the surface IDs has a nominal effect on the performance of the present techniques. FIG. 8B shows the performance of the present technique when patch sizes of 9 to 12 Å are used for a variety of features. As shown, an almost consistent AUC of 0.8 is achieved for when different features are used. The example features shown in FIG. 8B are curvature ("cv"), Charge ("ch"), hydrogen bond ("hb"), hydropathy ("hp"), and combinations of cv, ch, hb, and hp.

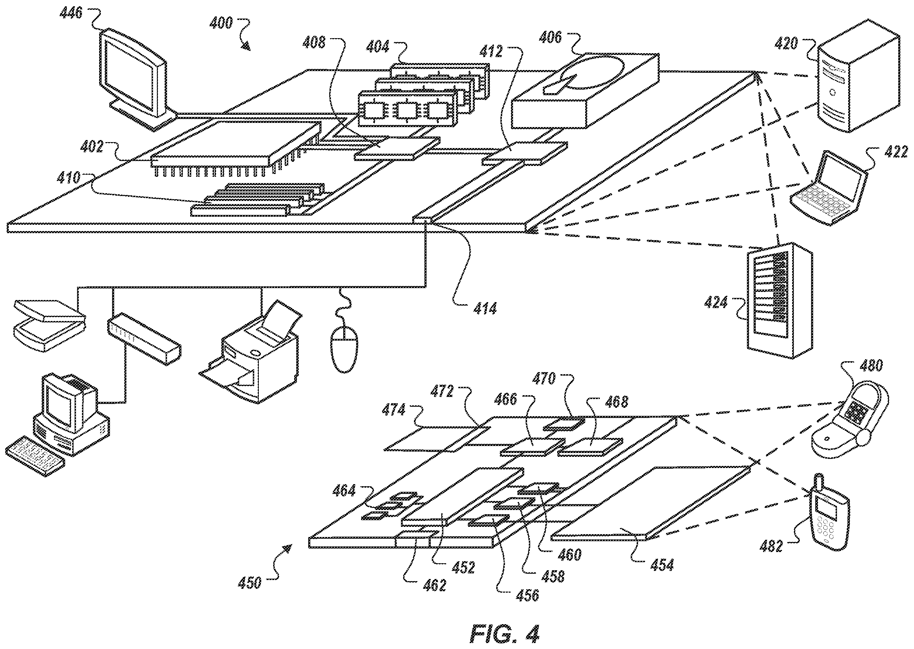

FIG. 4 shows an example of a computing device 400 and an example of a mobile computing device that can be used to implement the techniques described here. For example, system 100 in FIG. 1 can be the computing device 400, and the presentation device 120 can be the mobile computing device 480/482. The computing device 400 is intended to represent various forms of digital computers, such as laptops, desktops, workstations, personal digital assistants, servers, blade servers, mainframes, and other appropriate computers. The mobile computing device is intended to represent various forms of mobile devices, such as personal digital assistants, cellular telephones, smart-phones, and other similar computing devices. The components shown here, their connections and relationships, and their functions, are meant to be exemplary only, and are not meant to limit implementations of the inventions described and/or claimed in this document.

The computing device 400 includes a processor 402, a memory 404, a storage device 406, a high-speed interface 408 connecting to the memory 404 and multiple high-speed expansion ports 410, and a low-speed interface 412 connecting to a low-speed expansion port 414 and the storage device 406. Each of the processor 402, the memory 404, the storage device 406, the high-speed interface 408, the high-speed expansion ports 410, and the low-speed interface 412, are interconnected using various busses, and can be mounted on a common motherboard or in other manners as appropriate. The processor 402 can process instructions for execution within the computing device 400, including instructions stored in the memory 404 or on the storage device 406 to display graphical information for a GUI on an external input/output device, such as a display 416 coupled to the high-speed interface 408. In other implementations, multiple processors and/or multiple buses can be used, as appropriate, along with multiple memories and types of memory. Also, multiple computing devices can be connected, with each device providing portions of the necessary operations (e.g., as a server bank, a group of blade servers, or a multi-processor system).

The memory 404 stores information within the computing device 400. In some implementations, the memory 404 is a volatile memory unit or units. In some implementations, the memory 404 is a non-volatile memory unit or units. The memory 404 can also be another form of computer-readable medium, such as a magnetic or optical disk.

The storage device 406 is capable of providing mass storage for the computing device 400. In some implementations, the storage device 406 can be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product can also contain instructions that, when executed, perform one or more methods, such as those described above. The computer program product can also be tangibly embodied in a computer- or machine-readable medium, such as the memory 404, the storage device 406, or memory on the processor 402.

The high-speed interface 408 manages bandwidth-intensive operations for the computing device 400, while the low-speed interface 412 manages lower bandwidth-intensive operations. Such allocation of functions is exemplary only. In some implementations, the high-speed interface 408 is coupled to the memory 404, the display 416 (e.g., through a graphics processor or accelerator), and to the high-speed expansion ports 410, which can accept various expansion cards (not shown). In the implementation, the low-speed interface 412 is coupled to the storage device 406 and the low-speed expansion port 414. The low-speed expansion port 414, which can include various communication ports (e.g., USB, Bluetooth, Ethernet, wireless Ethernet) can be coupled to one or more input/output devices, such as a keyboard, a pointing device, a scanner, or a networking device such as a switch or router, e.g., through a network adapter.

The computing device 400 can be implemented in a number of different forms, as shown in the figure. For example, it can be implemented as a standard server 420, or multiple times in a group of such servers. In addition, it can be implemented in a personal computer such as a laptop computer 422. It can also be implemented as part of a rack server system 424. Alternatively, components from the computing device 400 can be combined with other components in a mobile device (not shown), such as a mobile computing device 450. Each of such devices can contain one or more of the computing device 400 and the mobile computing device 450, and an entire system can be made up of multiple computing devices communicating with each other.

The mobile computing device 450 includes a processor 452, a memory 464, an input/output device such as a display 454, a communication interface 466, and a transceiver 468, among other components. The mobile computing device 450 can also be provided with a storage device, such as a micro-drive or other device, to provide additional storage. Each of the processor 452, the memory 464, the display 454, the communication interface 466, and the transceiver 468, are interconnected using various buses, and several of the components can be mounted on a common motherboard or in other manners as appropriate.

The processor 452 can execute instructions within the mobile computing device 450, including instructions stored in the memory 464. The processor 452 can be implemented as a chipset of chips that include separate and multiple analog and digital processors. The processor 452 can provide, for example, for coordination of the other components of the mobile computing device 450, such as control of user interfaces, applications run by the mobile computing device 450, and wireless communication by the mobile computing device 450.

The processor 452 can communicate with a user through a control interface 458 and a display interface 456 coupled to the display 454. The display 454 can be, for example, a TFT (Thin-Film-Transistor Liquid Crystal Display) display or an OLED (Organic Light Emitting Diode) display, or other appropriate display technology. The display interface 456 can comprise appropriate circuitry for driving the display 454 to present graphical and other information to a user. The control interface 458 can receive commands from a user and convert them for submission to the processor 452. In addition, an external interface 462 can provide communication with the processor 452, so as to enable near area communication of the mobile computing device 450 with other devices. The external interface 462 can provide, for example, for wired communication in some implementations, or for wireless communication in other implementations, and multiple interfaces can also be used.

The memory 464 stores information within the mobile computing device 450. The memory 464 can be implemented as one or more of a computer-readable medium or media, a volatile memory unit or units, or a non-volatile memory unit or units. An expansion memory 474 can also be provided and connected to the mobile computing device 450 through an expansion interface 472, which can include, for example, a SIMM (Single In Line Memory Module) card interface. The expansion memory 474 can provide extra storage space for the mobile computing device 450, or can also store applications or other information for the mobile computing device 450. Specifically, the expansion memory 474 can include instructions to carry out or supplement the processes described above, and can include secure information also. Thus, for example, the expansion memory 474 can be provide as a security module for the mobile computing device 450, and can be programmed with instructions that permit secure use of the mobile computing device 450. In addition, secure applications can be provided via the SIMM cards, along with additional information, such as placing identifying information on the SIMM card in a non-hackable manner.

The memory can include, for example, flash memory and/or NVRAM memory (non-volatile random access memory), as discussed below. In some implementations, a computer program product is tangibly embodied in an information carrier. The computer program product contains instructions that, when executed, perform one or more methods, such as those described above. The computer program product can be a computer- or machine-readable medium, such as the memory 464, the expansion memory 474, or memory on the processor 452. In some implementations, the computer program product can be received in a propagated signal, for example, over the transceiver 468 or the external interface 462.

The mobile computing device 450 can communicate wirelessly through the communication interface 466, which can include digital signal processing circuitry where necessary. The communication interface 466 can provide for communications under various modes or protocols, such as GSM voice calls (Global System for Mobile communications), SMS (Short Message Service), EMS (Enhanced Messaging Service), or MMS messaging (Multimedia Messaging Service), CDMA (code division multiple access), TDMA (time division multiple access), PDC (Personal Digital Cellular), WCDMA (Wideband Code Division Multiple Access), CDMA2000, or GPRS (General Packet Radio Service), among others. Such communication can occur, for example, through the transceiver 468 using a radio-frequency. In addition, short-range communication can occur, such as using a Bluetooth, WiFi, or other such transceiver (not shown). In addition, a GPS (Global Positioning System) receiver module 470 can provide additional navigation- and location-related wireless data to the mobile computing device 450, which can be used as appropriate by applications running on the mobile computing device 450.

The mobile computing device 450 can also communicate audibly using an audio codec 460, which can receive spoken information from a user and convert it to usable digital information. The audio codec 460 can likewise generate audible sound for a user, such as through a speaker, e.g., in a handset of the mobile computing device 450. Such sound can include sound from voice telephone calls, can include recorded sound (e.g., voice messages, music files, etc.) and can also include sound generated by applications operating on the mobile computing device 450.

The mobile computing device 450 can be implemented in a number of different forms, as shown in the figure. For example, it can be implemented as a cellular telephone 480. It can also be implemented as part of a smart-phone 482, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms machine-readable medium and computer-readable medium refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term machine-readable signal refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing system that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network (LAN), a wide area network (WAN), and the Internet.

The computing system can include clients and servers. A client and server are generally remote from each other and typically interact through a communication network. The relationship of client and server arises by virtue of computer programs running on the respective computers and having a client-server relationship to each other.

## Claims

1. A method comprising:
receiving, by a system (100) of one or more computers, a target molecule (102) to be identified or classified;
identifying, by the system, a surface mesh that defines a surface of the target molecule, the surface mesh comprising a plurality of vertices;
identifying, by the system, a plurality of surface patches (102a, 102b) by associating each vertex of the surface mesh with a respective patch;
generating, by the system, a respective latent space representation (112a, 112b) for each of the surface patches by using a neural network;
generating, by the system, a respective real space representation (114a, 114b) for each patch in the surface patches by using a radial distribution of one or more geometric or chemical features of the patch;
obtaining, by the system and from a storage medium, one or more candidate items (208) with known surfaces;
using, by the system, the latent space representations to identify at least one candidate item (208k) that includes a surface resembling a surface region of the target molecule, wherein the surface region comprises multiple patches in the plurality of surface patches of the target molecule;
using, by the system, the real space representations to identify at least one candidate item (208n) that includes a surface resembling a surface region of the target molecule, wherein the surface region comprises multiple patches in the plurality of surface patches of the target molecule;
using the candidate items (208k, 208n) to determine an identification or a classification of the target molecule; and
providing, by the system, the identification or the classification of the target molecule for presentation to a user.

2. The method of claim 1, wherein identifying a first candidate item that includes a first surface resembling the surface region of the target molecule comprises:
mapping vertices of the target molecule to vertices of the first candidate item, wherein a first vertex on the target molecule is mapped to a second vertex on the first candidate item when a difference between at least one geometric or chemical feature at the first vertex and at the second vertex is within a predetermined threshold;
identifying a cluster of vertices on the target molecule that are each within a predetermined threshold distance from at least one of the mapped vertices on the target molecule;
aligning the cluster on the target molecule with multiple vertices of the first candidate item by using gradient descent, the multiple vertices being within the first surface on the first candidate item;
identifying, as the surface region, surface patches associated with the vertices of the cluster on the target molecule; and
providing the first candidate item as an item that includes the first surface resembling the surface region of the target molecule.

3. The method of claim 2, further comprising:
determining a spatial similarity score for the cluster based on a 3D distance between the vertices of the cluster and vertices on the first surface of the first candidate item; and
wherein the first candidate item is provided in response to determining that the spatial similarity score is within a threshold score.

4. The method of claim 2, further comprising:
identifying multiple clusters of vertices on the target molecule with vertices mapped to vertices of one or more candidate items; and
performing the method for each of the clusters to identify one or more surfaces on the one or more candidate items as resembling respective surface regions of the target molecule.

5. The method of claim 4, further comprising filtering out, from the multiple clusters, clusters that have less than a predetermined number of vertices.

6. The method of claim 4, further comprising:
ranking each cluster in the multiple clusters based on one or more of (i) number of mapped vertices in the cluster, (ii) a ratio of the number of mapped vertices in the cluster to a total number of vertices in the cluster, (iii) the number of vertices on the at least one candidate item mapped to one or more vertices of the cluster, and (iv) a ratio of the number of vertices on the at least one candidate item mapped to one or more vertices of the cluster, to a total number of vertices on the candidate item; and
filtering out, from multiple clusters, clusters that are ranked lower than a specific threshold rank.

7. The method of claim 2, wherein the at least one geometric or chemical feature at a vertex includes one or more of a shape index, a distance-dependent curvature, a hydropath, a continuum electrostatics, and a number of free electrons/protons at that vertex.

8. The method of claim 1, wherein the target molecule is a protein molecule, and a candidate item is a portion of a known protein molecule.

9. The method of claim 1, wherein the target molecule is an antigen, and a candidate item is an epitope, and wherein the method further comprises
using the identification or the classification of the antigen to design or identify an antibody for the antigen based on the epitope.

10. The method of claim 1, wherein the neural network comprises multiple layers, and each of the latent space representations is generated using the same layers.

11. A non-transitory, computer-readable medium storing one or more instructions executable by a computer system to perform operations comprising the method of any one of claims 1-10.

## Patentansprüche

1. Verfahren, das Folgendes umfasst:
Empfangen, durch ein System (100) eines oder mehrerer Computer, eines zu identifizierenden oder zu klassifizierenden Zielmoleküls (102);
Identifizieren, durch das System, eines Oberflächennetzes, das eine Oberfläche des Zielmoleküls definiert, wobei das Oberflächennetz eine Vielzahl von Knotenpunkten umfasst;
Identifizieren, durch das System, einer Vielzahl von Oberflächen-Patches (102a, 102b) durch Zuordnen jedes Knotenpunkts des Oberflächennetzes zu einem jeweiligen Patch;
Erzeugen, durch das System, einer jeweiligen Darstellung (112a, 112b) im latenten Raum für jeden der Oberflächen-Patches unter Verwendung eines neuronalen Netzes;
Erzeugen, durch das System, einer jeweiligen Darstellung (114a, 114b) im realen Raum für jeden Patch in den Oberflächen-Patches unter Verwendung einer radialen Verteilung eines oder mehrerer geometrischer oder chemischer Merkmale des Patches;
Erhalten, durch das System und von einem Speichermedium, eines oder mehrerer Kandidatenelemente (208) mit bekannten Oberflächen;
Verwenden, durch das System, der Darstellungen im latenten Raum zum Identifizieren mindestens eines Kandidatenelements (208k), das eine Oberfläche, die einer Oberflächenregion des Zielmoleküls ähnelt, aufweist, wobei die Oberflächenregion mehrere Patches in der Vielzahl von Oberflächen-Patches des Zielmoleküls umfasst;
Verwenden, durch das System, der Darstellungen im realen Raum zum Identifizieren mindestens eines Kandidatenelements (208n), das eine Oberfläche, die einer Oberflächenregion des Zielmoleküls ähnelt, aufweist, wobei die Oberflächenregion mehrere Patches in der Vielzahl von Oberflächen-Patches des Zielmoleküls umfasst;
Verwenden der Kandidatenelemente (208k, 208n) zum Bestimmen einer Identifizierung oder einer Klassifizierung des Zielmoleküls; und
Bereitstellen, durch das System, der Identifizierung oder der Klassifizierung des Zielmoleküls zur Präsentation für einen Benutzer.

2. Verfahren nach Anspruch 1, wobei das Identifizieren eines ersten Kandidatenelements, das eine erste Oberfläche, die der Oberflächenregion des Zielmoleküls ähnelt, aufweist, Folgendes umfasst:
Abbilden von Knotenpunkten des Zielmoleküls auf Knotenpunkte des ersten Kandidatenelements, wobei ein erster Knotenpunkt auf dem Zielmolekül auf einen zweiten Knotenpunkt auf dem ersten Kandidatenelement abgebildet wird, wenn eine Differenz zwischen mindestens einem geometrischen oder chemischen Merkmal an dem ersten Knotenpunkt und an dem zweiten Knotenpunkt innerhalb eines vorbestimmten Schwellenwerts liegt;
Identifizieren eines Clusters von Knotenpunkten auf dem Zielmolekül, die sich jeweils innerhalb eines vorbestimmten Schwellenwertabstands von mindestens einem der abgebildeten Knotenpunkte auf dem Zielmolekül befinden;
Ausrichten des Clusters auf dem Zielmolekül auf mehrere Knotenpunkte des ersten Kandidatenelements unter Verwendung eines Gradientenabstiegs, wobei sich die mehreren Knotenpunkte innerhalb der ersten Oberfläche auf dem ersten Kandidatenelement befinden;
Identifizieren, als die Oberflächenregion, von den Knotenpunkten des Clusters auf dem Zielmolekül zugeordneten Oberflächen-Patches; und
Bereitstellen des ersten Kandidatenelements als ein Element, das die erste Oberfläche, die der Oberflächenregion des Zielmoleküls ähnelt, aufweist.

3. Verfahren nach Anspruch 2, das ferner Folgendes umfasst:
Bestimmen eines räumlichen Ähnlichkeitswerts für den Cluster basierend auf einem 3D-Abstand zwischen den Knotenpunkten des Clusters und Knotenpunkten auf der ersten Oberfläche des ersten Kandidatenelements; und
wobei das erste Kandidatenelement als Reaktion auf das Bestimmen, dass der räumliche Ähnlichkeitswert innerhalb eines Schwellenwertscores liegt, bereitgestellt wird.

4. Verfahren nach Anspruch 2, das ferner Folgendes umfasst:
Identifizieren mehrerer Cluster von Knotenpunkten auf dem Zielmolekül mit Knotenpunkten, die auf Knotenpunkte eines oder mehrerer Kandidatenelemente abgebildet sind; und
Durchführen des Verfahrens für jeden der Cluster, um eine oder mehrere Oberflächen auf dem einen oder den mehreren Kandidatenelementen als jeweiligen Oberflächenregionen des Zielmoleküls ähnelnd zu identifizieren.

5. Verfahren nach Anspruch 4, das ferner das Herausfiltern von Clustern, die weniger als eine vorbestimmte Anzahl von Knotenpunkten aufweisen, aus den mehreren Clustern umfasst.

6. Verfahren nach Anspruch 4, das ferner Folgendes umfasst:
Einstufen jedes Clusters in den mehreren Clustern basierend auf (i) einer Anzahl abgebildeter Knotenpunkte in dem Cluster und/oder (ii) einem Verhältnis der Anzahl abgebildeter Knotenpunkte in dem Cluster zu einer Gesamtzahl von Knotenpunkten in dem Cluster und/oder (iii) der Anzahl von Knotenpunkten auf dem mindestens einen Kandidatenelement, die auf einen oder mehrere Knotenpunkte des Clusters abgebildet sind, und/oder (iv) einem Verhältnis der Anzahl von Knotenpunkten auf dem mindestens einen Kandidatenelement, die auf einen oder mehrere Knotenpunkte des Clusters abgebildet ist, zu einer Gesamtzahl von Knotenpunkten auf dem Kandidatenelement; und
Herausfiltern von Clustern, die niedriger als ein bestimmter Schwellenwertrang eingestuft sind, aus mehreren Clustern.

7. Verfahren nach Anspruch 2, wobei das mindestens eine geometrische oder chemische Merkmal an einem Knotenpunkt einen Formindex und/oder eine abstandsabhängige Krümmung und/oder eine Hydropathie und/oder eine Kontinuumselektrostatik und/oder eine Anzahl freier Elektronen/Protonen an diesem Knotenpunkt aufweist.

8. Verfahren nach Anspruch 1, wobei das Zielmolekül ein Proteinmolekül ist und ein Kandidatenelement ein Abschnitt eines bekannten Proteinmoleküls ist.

9. Verfahren nach Anspruch 1, wobei das Zielmolekül ein Antigen ist und ein Kandidatenelement ein Epitop ist und wobei das Verfahren ferner Folgendes umfasst:
Verwenden der Identifizierung oder der Klassifizierung des Antigens zum Entwerfen oder Identifizieren eines Antikörpers für das Antigen basierend auf dem Epitop.

10. Verfahren nach Anspruch 1, wobei das neuronale Netz mehrere Schichten umfasst und jede der Darstellungen im latenten Raum unter Verwendung der gleichen Schichten erzeugt wird.

11. Nichttransitorisches, computerlesbares Medium, das eine oder mehrere Anweisungen speichert, die durch ein Computersystem ausführbar sind, um Vorgänge, die das Verfahren nach einem der Ansprüche 1-10 umfassen, durchzuführen.

## Revendications

1. Procédé, comprenant :
la réception, par un système (100) d'un ou plusieurs ordinateurs, d'une molécule cible (102) à identifier ou à classifier ;
l'identification, par le système, d'un maillage de surface qui définit une surface de la molécule cible, le maillage de surface comprenant une pluralité de sommets ;
l'identification, par le système, d'une pluralité de carreaux de surface (102a, 102b) en associant chaque sommet du maillage de surface à un carreau respectif ;
la génération, par le système, d'une représentation d'espace latent (112a, 112b) respective pour chacun des carreaux de surface en utilisant un réseau neuronal ;
la génération, par le système, d'une représentation de l'espace réel (114a, 114b) respective pour chaque carreau dans les carreaux de surface en utilisant une distribution radiale d'une ou plusieurs caractéristiques géométriques ou chimiques du carreau ;
l'obtention, par le système et à partir d'un support de stockage, d'un ou plusieurs éléments candidats (208) ayant des surfaces connues ;
l'utilisation, par le système, des représentations d'espace latent pour identifier au moins un élément candidat (208k) qui comprend une surface ressemblant à une zone de surface de la molécule cible, la zone de surface comprenant plusieurs carreaux dans la pluralité de carreaux de surface de la molécule cible ;
l'utilisation, par le système, des représentations spatiales réelles pour identifier au moins un élément candidat (208n) qui comprend une surface ressemblant à une zone de surface de la molécule cible, la zone de surface comprenant de multiples carreaux dans la pluralité de carreaux de surface de la molécule cible ;
l'utilisation des éléments candidats (208k, 208n) pour déterminer une identification ou une classification de la molécule cible ; et
la fourniture, par le système, de l'identification ou de la classification de la molécule cible pour une présentation à un utilisateur.

2. Procédé selon la revendication 1, dans lequel l'identification d'un premier élément candidat qui comprend une première surface ressemblant à la zone de surface de la molécule cible comprend :
le mappage de sommets de la molécule cible sur des sommets du premier élément candidat, un premier sommet sur la molécule cible étant mappé sur un second sommet sur le premier élément candidat lorsqu'une différence entre au moins une caractéristique géométrique ou chimique au niveau du premier sommet et au niveau du second sommet se situe à l'intérieur d'un seuil prédéterminé ;
l'identification d'un cluster de sommets sur la molécule cible qui se trouvent chacun à une distance seuil prédéterminée d'au moins un des sommets cartographiés sur la molécule cible ;
l'alignement du cluster sur la molécule cible avec plusieurs sommets du premier élément candidat en utilisant une descente de gradient, les multiples sommets se trouvant à l'intérieur de la première surface sur le premier élément candidat ;
l'identification, en tant que zone de surface, de carreaux de surface associés aux sommets du cluster sur la molécule cible ; et
la fourniture du premier élément candidat en tant qu'élément qui comprend la première surface ressemblant à la zone de surface de la molécule cible.

3. Procédé selon la revendication 2, comprenant en outre :
la détermination d'un score de similarité spatiale pour le cluster sur la base d'une distance en 3D entre les sommets du cluster et les sommets sur la première surface du premier élément candidat ; et
le premier élément candidat étant fourni en réponse à la détermination du fait que le score de similarité spatiale se situe à l'intérieur d'un score seuil.

4. Procédé selon la revendication 2, comprenant en outre :
l'identification de plusieurs clusters de sommets sur la molécule cible ayant des sommets mappés sur les sommets d'un ou plusieurs éléments candidats ; et
la mise en œuvre du procédé pour chacun des clusters afin d'identifier une ou plusieurs surfaces sur le ou les éléments candidats comme ressemblant à des zones de surface respectives de la molécule cible.

5. Procédé selon la revendication 4, comprenant en outre le filtrage, à partir des multiples clusters, des clusters qui ont moins d'un nombre prédéterminé de sommets.

6. Procédé selon la revendication 4, comprenant en outre :
le classement de chaque cluster dans les multiples clusters sur la base d'un ou plusieurs parmi (i) un nombre de sommets mappés dans le cluster, (ii) un rapport du nombre de sommets mappés dans le cluster sur un nombre total de sommets dans le cluster, (iii) le nombre de sommets sur le ou les éléments candidats mappés sur un ou plusieurs sommets du cluster, et (iv) un rapport entre le nombre de sommets sur le ou les éléments candidats mappés sur un ou plusieurs sommets du cluster, et un nombre total de sommets sur l'élément candidat ; et
le filtrage, à partir de multiples clusters, de clusters qui sont classés en dessous d'un rang de seuil spécifique.

7. Procédé selon la revendication 2, dans lequel l'au moins une caractéristique géométrique ou chimique au niveau d'un sommet comprend un ou plusieurs parmi un indice de forme, une courbure dépendant de la distance, une hydropathie, une électrostatique de continuum et un nombre d'électrons/protons libres au niveau de ce sommet.

8. Procédé selon la revendication 1, dans lequel la molécule cible est une molécule de protéine, et un élément candidat est une partie d'une molécule de protéine connue.

9. Procédé selon la revendication 1, dans lequel la molécule cible est un antigène, et un élément candidat est un épitope, et dans lequel le procédé comprend en outre
l'utilisation de l'identification ou de la classification de l'antigène pour concevoir ou identifier un anticorps pour l'antigène basé sur l'épitope.

10. Procédé selon la revendication 1, dans lequel le réseau neuronal comprend plusieurs couches, et chacune des représentations d'espace latent est générée en utilisant les mêmes couches.

11. Support non transitoire lisible par ordinateur stockant une ou plusieurs instructions exécutables par un système informatique pour mettre en œuvre des opérations comprenant le procédé selon l'une quelconque des revendications 1 à 10.
